# EUROPEAN PATENT SPECIFICATION

(11) **EP 3 284 413 B1**
(45) Date of publication and mention of the grant of the patent: **18.12.2019**
(21) Application number: 16184290.1
(22) Date of filing: 16.08.2016
(51) Int. Cl.: A61B 17/068, A61F 2/24, A61B 17/128

(54) **MEDICAL SECURING DEVICE FOR SECURING A CARDIAC IMPLANT DEVICE WITH A SECURING MEMBER**
MEDIZINISCHE SICHERUNGSVORRICHTUNG ZUR SICHERUNG EINER HERZIMPLANTATVORRICHTUNG MIT EINEM BEFESTIGUNGSELEMENT
DISPOSITIF DE FIXATION MÉDICALE PERMETTANT DE FIXER UN DISPOSITIF D'IMPLANT CARDIAQUE AVEC UN ÉLÉMENT DE FIXATION

(43) Date of publication of application: 21.02.2018
(73) Proprietor: Medtentia International Ltd Oy, 02600 Espoo (FI)
(72) Inventor: ALLEN, William, 02600 Espoo (FI); BACHMAN, Allan, 02600 Espoo (FI); LEHMAN, Adam, 02600 Espoo (FI); TYNNERSTÅL, Jonas, 02600 Espoo (FI); KERÄNEN, Olli, 02600 Espoo (FI)
(74) Representative: Berggren Oy, Helsinki & Oulu

(56) References cited:
- WO-A1-2015/124630
- US-A1- 2009 188 964
- US-A1- 2011 301 701
- US-A1- 2016 045 315

## Description

### TECHNICAL FIELD OF THE INVENTION

The invention relates to a medical securing device for securing a cardiac implant (annuloplasty medical) device with a securing member. In particularly the invention relates to a medical securing device for an open-heart-operation and for securing the cardiac implant device into an annulus of a heart valve, such as a mitral valve or tricuspid valve, comprised of valve tissue and including the annulus and a plurality of leaflets.

### BACKGROUND OF THE INVENTION

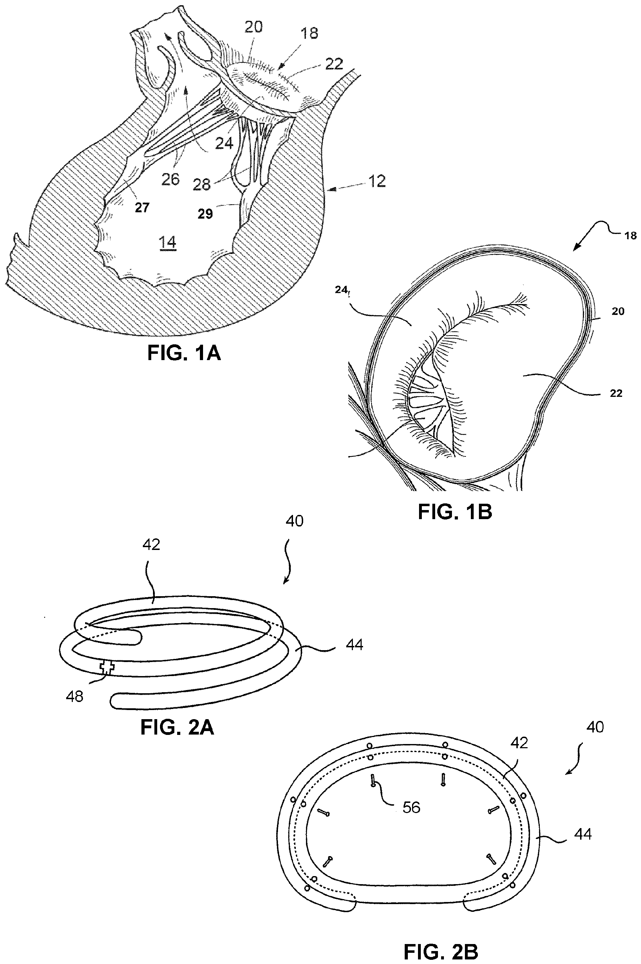

Fig. 1A illustrates a portion of the heart 12, the mitral valve 18, and the left ventricle 14. The mitral valve is at its boundary circumferenced by an annulus 20. The valve has two cusps or leaflets 22, 24. Each of these cusps or leaflets 22, 24 are connected to a respective papillary muscle 27, 29 via their respective connecting chordae 26, 28. In normal healthy individuals the free edges of the opposing leaflets will close the valve by coaptation. However, for some individuals the closure is not complete, which results in a regurgitation, also called valvular insufficiency, i.e. back flow of blood to the left atrium making the heart less effective and with potentially severe consequences for the patient. Fig. 1B illustrates a mitral valve 18, in which the leaflets 22, 24 do not close properly. This commonly occurs when the annulus 20 becomes dilated. One surgical procedure to correct this is to remove a portion of the leaflet 24 and stitch the cut edges together with one another. The procedure will pull back the annulus 20 to a more normal position. However the strength of the leaflet 24 is altered. Similar problems with a less effective heart function occur if one or both leaflets are perforated to such an extent that blood is flowing towards the left atrium, although the leaflets close properly.

In some conditions of degenerated heart function, the leaflets do not present a solid surface, as in a degenerative valve disease. The leaflet may also be ruptured, most commonly at an edge of a leaflet, resulting in an incomplete coaptation. Hence, cardiac devices and methods are developed for repairing of one or more leaflets of a heart valve, or other related anatomical structures, such as the chordae attached to the ventricular side of leaflets.

Fig. 2A-B illustrate prior art cardiac implant devices and method for repairing of one or more leaflets of a heart valve as is described in the applicant's previous EP-patent (EP 1 853 199 B1), where the device 40 comprises a first and a second loop-shaped support 42, 44, which are connected to each other by means of a connecting part 48 so as to form a coil-shape. The coil-shape of the device is advantageous during insertion, since the device 40 may then be rotated into position, as described in the patent in more details. One of the supports 44 may be open, e.g. C or D or any other anatomical shaped such that the support 44 presents an end to lead the movement of the support 44 when being rotated into position. The position of the supports 42, 44 are secured by fasteners 156, which are inserted and fastened by hand or small screwdriver.

It is found that the prior art cardiac implant devices, such as depicted above, work very well, but there are still some disadvantages relating to the securing of the cardiac device into the annulus of the heart valve. The cardiac devices are typically sutured by a needle and yarns, which is time consuming, because in practise it is needed at least seven knots to be tied in order to have even some certainty that the device is secured. In addition, the device is sutured typically by one yarn, which has a drawback namely if one or more knots is/are loosen or the yarn is broken, then the whole securing will come loosen or broken.

The cardiac implant devices are also secured by screws. However, the screws are very small, the assembling, positioning and controlling of which are extremely difficult. The screws must be inserted through the both the first and second (upper and lower) loop-shaped support portions 42, 44 (tiny holes in both of the portions), which is highly demanding, because if the first screw is tightened too much, it will distort the portions little bit and thus misaligning the other holes and thereby making it impossible to inserting the other screws. Furthermore there is a huge risk to drop the small screws into the cardiac structure, because for example any safety blankets cannot be used. In addition also magnetic material cannot be used due to possible later magnetic imaging.

In addition WO2015/124630A1 discloses a stapling device for attaching a clip to tissue comprising a sheath having a distal end for delivery of said clip, a pusher unit being movable inside said sheath along a longitudinal direction of said sheath, said distal end comprising a clip guide in which said clip is movable in said longitudinal direction, wherein said clip guide has a closed configuration in which said clip guide is adapted to apply a restraining force on said clip so that said clip assumes a delivery shape, and an open configuration in which said clip assumes a relaxed shape, wherein said pusher unit is movable from a proximal position in which said clip guide is in said closed configuration, to a distal position in which said pusher unit engages said clip guide and the clip guide is in said open configuration. The clip guide comprises a first and a second clip guide arranged at radially opposite peripheries of the sheath and extending in the longitudinal direction.

US2016/045315A1 in turn discloses a device to be positioned in a sealed introducer arranged in the thoracic cavity between two ribs in order to penetrate into the left ventricle, passing through the apex of the heart (Device For Carrying Out A Transapical Mitral Valve Annuloplasty). The device includes a control member capable of acting on an assembly for installing and securing a braid to the mitral annulus by means of suturing elements, the assembly has a mechanism capable of enabling the extraction of a suture through the mitral valve while also being capable of enclosing the braid and becoming anchored to the periphery of the mitral annulus under the clamping effect of the suture, exerting two opposing pressure-bearing forces. The device has first and second arm portion, where the second arm portion is made of several members jointed and mounted movable and steerable relative to the first arm.

### SUMMARY OF THE INVENTION

It is an object of the invention to alleviate and eliminate the problems relating to the known prior art. Especially the object of the invention is to provide a medical securing device for securing a cardiac implant device with a securing member into an annulus of a heart valve in an easy, fast and safe manner.

The object of the invention can be achieved by the features of independent claims.

The invention relates to a medical securing device for securing a cardiac implant device with a securing member into an annulus of a heart valve according to claim 1.

According to an embodiment of the invention a medical securing device for securing a cardiac implant device with a securing member into an annulus of a heart valve comprises at least a first elongated stem. The first stem has proximal and distal ends, and the distal end has a receiving member for receiving the securing member. The receiving member is advantageously a piston type member, which is configured to receive the securing member, such as pinch, press or lock the securing member mechanically. In addition the medical securing device is advantageously configured to under manipulation, such as during a movement of the distal end of the first stem and the receiving member in relation to each other, move, introduce and secure, such as press, bend, twist or otherwise secure the securing member to the cardiac implant device and thereby to secure the upper and/or lower portion of the cardiac implant device to the annulus of the valve with the securing member.

According to an embodiment the distal end of the first stem has a hollow structure, such as a conduit or channel, where the receiving member is arranged in a movable manner so that during the movement the receiving member moves and thereby introduces the securing member from the hollow structure to the cardiac implant device thereby securing the cardiac implant device to the annulus of the valve with the securing member.

In addition according to an embodiment the medical securing device may further comprise also a second elongated stem adjacent to the first stem. The first and second stems are configured to be moved advantageously under the same manipulation or movement in relation to each other in their longitudinal direction, both stems having proximal and distal ends. The distal end of the second stem has a counterpart portion, such as a clamp and/or anvil. The counterpart portion can be used together the distal end of the first stem for clamping the cardiac implant device between the counterpart portion and the distal end, but also together the receiving member for stapling the securing member to the cardiac implant device and/or to the annulus.

The distal end of the second stem is advantageously configured to be introduced to the opposite portion of the valve tissue than the distal end of the first stem or to the lower portion of the cardiac implant device, when lower portion is used. In addition the distal end is configured to produce counterforce via the counterpart portion to the distal end of the first stem, for example when the second stem is pulled, and thereby clamping the cardiac implant device between the distal ends of the first and second stems when the distal end of the first and/or second stems is/are moved in the longitudinal direction towards the cardiac implant device. In addition during the additional movement the distal end of the first stem with the receiving member or the receiving member as such is configured to introduce the securing member to the cardiac implant device to secure at least portion of the cardiac implant device to the annulus of the valve with the securing member.

The securing member may be e.g. a staple the one end of which is bendable at least partially around or into the cardiac implant device under the pressing force induced by the distal end of the first stem and/or counterpart portion of the second stem. The securing member may also be e.g. a staple having at least one hook-shaped end to be introduced at least partially around or into the cardiac implant device. In addition the securing member may be also a helical clip, locking clip, pointed screw, spring clip, skin staple, pin, or circular clip, as an example. The securing member may comprise for example shape memory material, metal or polymer or any other material with memory function, as an example. In addition the second end or both ends of the securing member may be sharpened (atraumatic) so to help the penetration of the securing member into the annulus tissue.

It is to be noted that the heart valve is for example a mitral valve or tricuspid valve, and comprises valve tissue including the annulus and a plurality of leaflets. In addition it is to be noted that the cardiac implant device may comprise an upper or lower portion, or both. The portions are advantageously as loops and at least one of them can be open loop, such as C or D shaped portion (or any other suitable anatomical shape), whereby, in use, a portion of the valve tissue is coupled with the upper or lower portion, or trapped between the upper and lower portions of the cardiac implant.

The present invention offers advantages over the known prior art, such as an easy, safe and time saving manner to reliable securing the cardiac implant device to the annulus of the valve with the securing member. In addition the securing process including both the clamping and positioning of the cardiac implant device, as well as the securing the cardiac implant device in a reliable manner into the right position can be performed in a very natural way and advantageously by one movement. Most advantageously the movement can be just one continuous movement, such as for example by pressing or pulling the operation member(s) in the handle bar of the medical securing device.

The exemplary embodiments presented in this text are not to be interpreted to pose limitations to the applicability of the appended claims. The verb "to comprise" is used in this text as an open limitation that does not exclude the existence of also un-recited features. The features recited in depending claims are mutually freely combinable unless otherwise explicitly stated.

The novel features which are considered as characteristic of the invention are set forth in particular in the appended claims. The invention itself, however, both as to its construction and its method of operation, together with additional objects and advantages thereof, will be best understood from the following description of specific example embodiments when read in connection with the accompanying drawings.

### BRIEF DESCRIPTION OF THE DRAWINGS

Next the invention will be described in greater detail with reference to exemplary embodiments in accordance with the accompanying drawings, in which:
- Figures 1A-1B: illustrate schematically a portion of a heart and mitral valve,
- Figures 2A-2B: illustrate a prior art cardiac implant device for repairing of one or more leaflets of a heart valve, and
- Figures 3A-13: illustrate examples of medical securing devices according to advantageous embodiments of the invention.

### DETAILED DESCRIPTION

Figures 1A-1B and 2A-2B are already discussed in more details in connection with the background of the invention portion above.

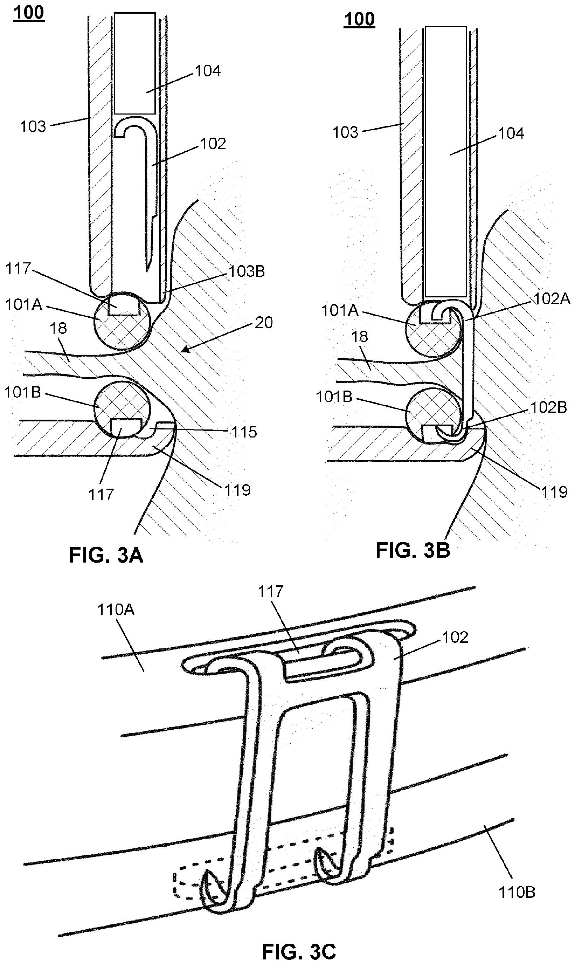

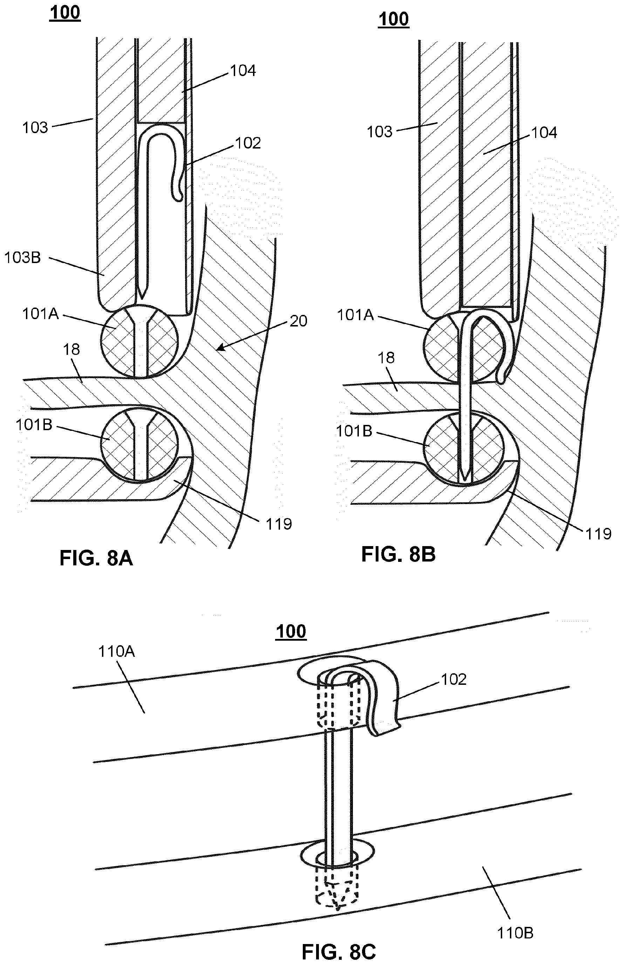

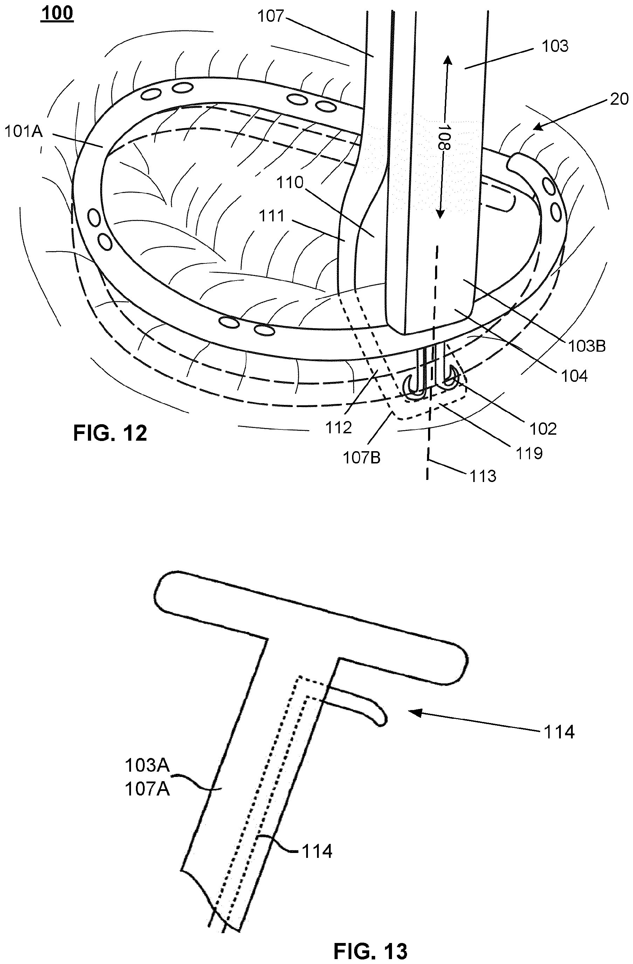

Figures 3A-13 illustrate examples of medical securing devices 100 according to advantageous embodiments of the invention, where the medical securing device 100 comprises at least a first elongated stem 103. The first stem has proximal 103A and distal 103B ends (Fig. 12), and the distal end 103B has a receiving member 104 for receiving the securing member 102. The distal end 103B of the first stem and the receiving member 104 can be moved in relation to each other, whereupon they are configured to introduce and secure the securing member 102 to the cardiac implant device 101 and thereby to secure the upper and/or lower portion 101A, 101B of the cardiac implant device 101 to the annulus 20 of the valve with the securing member 102.

In addition the medical securing device 100 may also comprise a second elongated stem 107 adjacent to the first stem, as is described in more details in Fig. 12, but also in Figures 3, 4, 6, 7, 8. The first 103 and second 107 stems are configured to be moved advantageously under the same manipulation or movement in relation to each other in their longitudinal direction 108. The distal end 107B of the second stem 107 is advantageously configured to be introduced to the opposite portion of the valve tissue than the distal end 103B of the first stem 103 or to the lower portion 101B of the cardiac implant device 101, when the cardiac implant device 101 comprises the lower portion 101B.

The distal end 107B of the second stem 107 has advantageously a counterpart portion 119, which can be used for clamping and thereby controlling the cardiac implant device 101 between the counterpart portion 119 and the distal end 103B of the first stem for further operation, namely for introduction the securing member 102. In addition the counterpart portion 119 can be used, together with the receiving member 104, for securing, such as stapling the securing member 102 to the cardiac implant device 101 and/or to the annulus 20. As can be seen in Figures the counterpart portion 119 can be used as an anvil to guide and bend the securing member 102 around or into the contact with the cardiac implant device 101 and/or to the annulus 20, when introduced by the receiving member 104.

Especially the distal end 107B is configured to produce counterforce via the counterpart portion 119 to the distal end 103B of the first stem 103, for example when the second stem 107 is pulled (107A, 114). For example the operation member illustrated in Fig. 13 can be arranged so that when the proximal end 103A, 107A of the first and/or second stem(s) 103, 107 and/or the operation member 114 are pressed against each other, the distal end of the first and/or second stems is/are moved in the longitudinal direction 108 towards the cardiac implant device 101, and the cardiac implant device 101 is then clamped between the distal ends 103B, 107B of the first and second stems 103, 107. In addition during the additional movement 108 the distal end 103B of the first stem 103 with the receiving member 104 or the receiving member 104 as such is configured to introduce the securing member 102 to the cardiac implant device 101 to secure at least portion of the cardiac implant device 101 to the annulus 20 of the valve with the securing member 102.

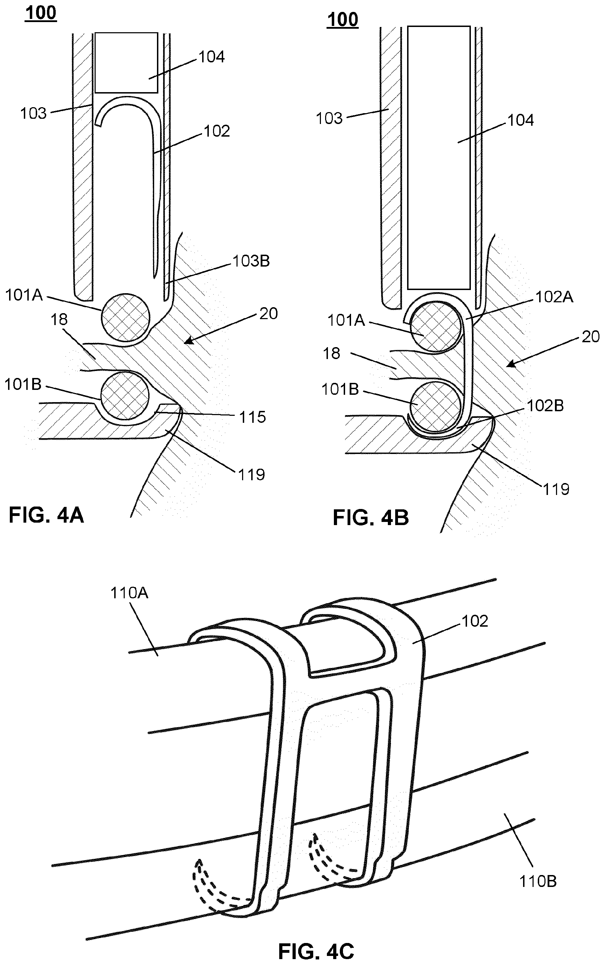

The distal end 103B of the first stem 103 or the receiving member 104 is configured to press and/or bend a first end 102A of the securing member at least partially around or partially into the upper and/or lower portion 101A, 101B of the cardiac implant device 101, when the distal end 103B of the first stem 103 and/or the receiving member 104 is pressed against the counterpart portion 119 of the second stem 107. As can be seen e.g. in Fig. 3A and 3B the ends 102A, 102B of the securing member 102 are pressed and bended into the slots 117 in the cardiac implant device 101. The securing member 102 may be as a nail or having H-form (see. e.g. Fig. 3C), and the first end 102A of it can have a hook shape originally, whereupon the second end 102B can be bend by the counterpart portion 119 functioning as an anvil. Alternatively the securing member 102 can be introduced around the upper and lower portions 101A, 101B of the cardiac implant device 101, as is illustrated in Fig. 4A-4C. Also in this example the first end 102A of the securing member 102 comprises a hook shape originally, and the second end 102B is formed and guided around the lower portion 101B of the cardiac implant device 101 by the counterpart portion 119 functioning as the anvil.

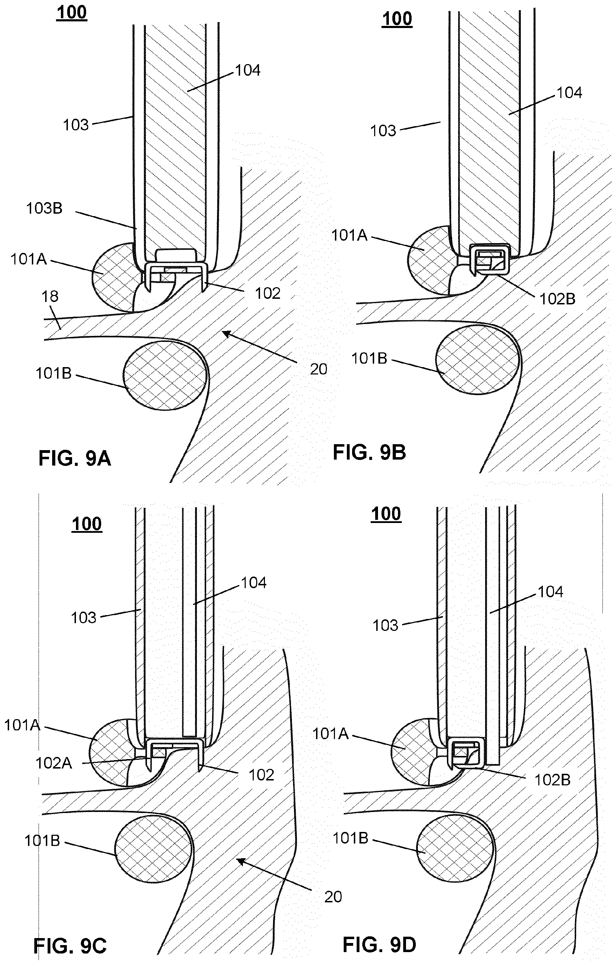

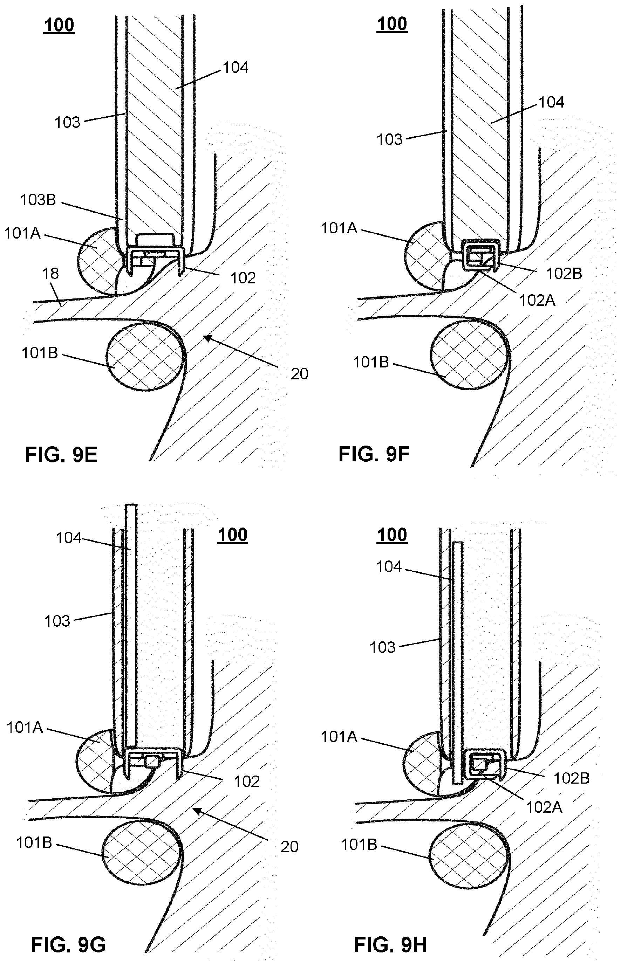

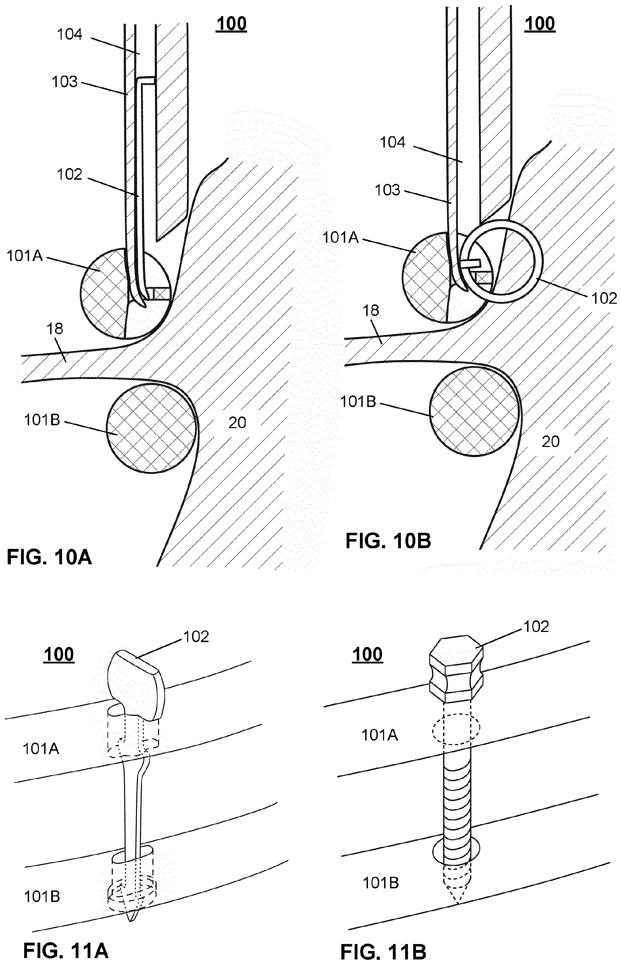

It also should be noted that the distal end 103B of the first stem 103 (advantageously with the receiving member (104)) as such or together with the counterpart portion 119 of the second stem 107 may also be configured to press and/or bend the second end 102B of the securing member 102 at least partially into the annulus 20 of the valve, as is described in Fig. 9A-9D, or Fig. 10A-10B. In particularly in Fig. 9A-9B the receiving member 104 can be arranged so that when it is pulled upwards it will raise the centre portion of the securing member 102 (as a staple), whereupon the ends of the securing member 102 will bend downwards and are thereby introduced into the cardiac implant device 101 and the annulus tissue 20. In Fig. 9C-9D the receiving member 104 (such as a piston type member) is pushed downwards, whereupon it will bend at least one end of the securing member 102 (as a staple) downwards and is thereby introduced into the cardiac implant device 101 and the annulus tissue 20. The securing member 102 may also comprise shape memory material, which additionally helps to achieve the form and securing of the securing member e.g. to the annulus as is described in Fig. 10A-10B.

It is to be noted that in the embodiment illustrated in Fig. 9A-9D the first end 102A of the securing member 102 is introduced into or coupled with the cardiac implant device 101 and the second end 102B of the securing member 102 is introduced into or coupled with the annulus tissue 20 (or bend via the annulus tissue towards or into the cardiac implant device 101). Again it is to be noted that in the embodiment illustrated in Fig. 9E-9H the first end 102A of the securing member 102 is introduced into or coupled with the cardiac implant device 101 and then bend towards and further into the annulus tissue 20, and the second end 102B of the securing member 102 is introduced directly to the the annulus tissue 20.

As can be seen in the embodiment and drawings, the present invention offer many advantageously features and ways to secure the cardiac implant device into the annulus. For example, the embodiment illustrated in Fig. 9E-9H offer clear advantage, namely at first the introduction of the securing member 102 as well as the securing of the cardiac implant 101 can be done by the medical securing device 100 having only the first stem 103 (either "upper" 103 or "lower" 107 stem. In this embodiment the distal end 103B of the first stem 103 (or 107) with the receiving member (104) is configured to introduce the securing device 102 at least partially through or via the cardiac implant device 101 into the annulus 20 of the valve during the movement of the distal end 103B of the first stem 103 (or 107) and/or the receiving member 104 in relation to the cardiac implant device 101. The movement can be e.g. downwards so towards the cardiac implant device 101 or upwards so away from the cardiac implant device 101.

However when the first end 102A of the securing member is 102 is introduced into or coupled with the cardiac implant device 101 and then bend towards and further into the annulus tissue 20 (and the second end 102B of the securing member 102 is introduced "directly" to the the annulus tissue 20), huge advantage can be achieved, namely when the end (102A) of the securing member 102 to be introduced into the annulus tissue 20 is bend before introduction, the tensile stress, as well as any other further tissue tearing caused to the tissue can be remarkably reduced.

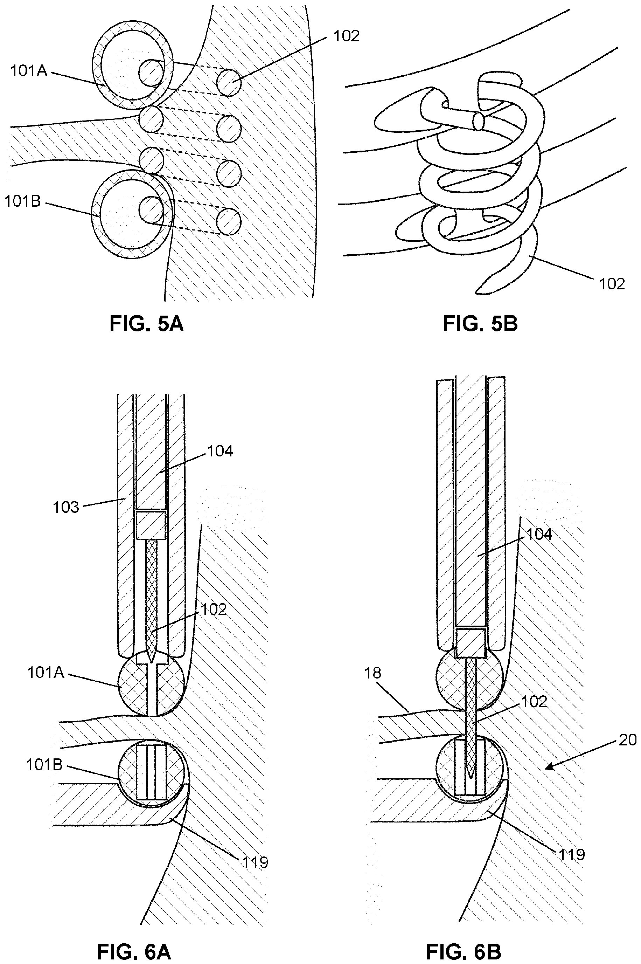

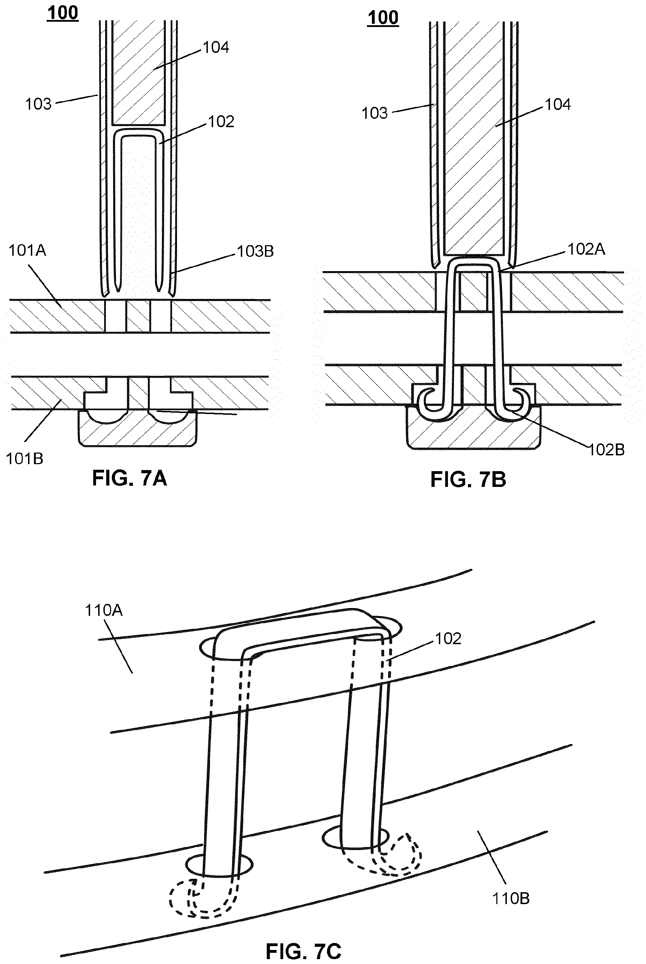

In addition it is to be noted that the securing member 102 may be as a locking clip or pointed screw or the like as is described in Fig. 11A, 11B, whereupon the receiving member 104 can be turned e.g. via manipulation of the operation member 114 so to introduce and turn (screw) the securing member 102 into the cardiac implant device 101. Moreover, as can be seen in Fig. 5A, 5B, the securing member 102 may be as a helical spring 102, whereupon the receiving member 104 possibly together the distal end 103B of the first stem 103 is configured to introduce the helical spring 102 so to penetrate a possible hollow or hole structure in the cardiac implant device 101 as well as to penetrate into the annulus 20 tissue of the valve structure. The cardiac implant device 101 may also comprise a textile structure around it to which the helical spring 102 can be secured. Still in Fig. 6A, 6B is shown a locking clip 102, which can be just pushed through the first hole in the upper portion of the cardiac implant device 101 and again into the receiving securing hole in the lower portion of the cardiac implant device 101, where the securing hole locks the locking clip 102 in its securing position. Additionally Fig. 7A-7C illustrates a hook shaped securing member 102 which is pressed by the receiving member 104 thought the holes in the upper portion 101A of the cardiac implant device 101 and again so that the second end 102B of the hook shaped securing member 102 is bend into the slots of the lower portion 101B of the cardiac implant device 101.

The distal end 103B of the first stem 103 may also comprise a recess 116 for supporting the distal end 103B of the first stem 103 to the upper portion 101A of the cardiac implant device 101. The distal end 107B of the second stem 107 may also comprise a recess 117 for supporting the distal end 107B of the second stem 107 to the lower portion 101B of the cardiac implant device 101 during the introduction of the securing member 102. This helps and guides the securing device 100 to be positioned in an appropriate position for clamping as well as for introducing the securing member and thereby for securing the cardiac implant device 101 into the annulus.

As can be seen especially in Fig. 12 the mutual design of the distal end portions 103B, 107B of the first and second stems 103, 107 are so that a space 110 is formed between the distal end portions. As an example, the space 111 the second stem 107 may comprise a protrusion portion 111 in the distal end portion 107B 110 between the first and second stems 103, 107. In a use the space 110 is configured to receive at least portion of the valve tissue and/or leaflets 18. In addition the distal end 107B of the second stem may have an angle 112, such as inclined or rectangular portion, which extends towards the extension line 113 of the distal end 103B of the first stem 103 so that the counterpart portion 119 of the second stem 107 locates essentially in the extension line 113 in the longitudinal direction 108 of the first stem distal end portion 103B.

The distal end 103B of the first stem 103 has a hollow structure 106, such as a conduit or channel, where the receiving member 104 is arranged in a movable manner. In addition the first stem 103 has a hollow structure 106 via which the operation member 114 is functionally coupled with the receiving member 104. During the movement the receiving member 104 moves and when it is moved towards the output of the distal end 103B, it thereby introduces the securing member from the hollow structure to the cardiac implant device 101 and secures the cardiac implant device (or at least part of it) to the annulus 20 of the valve with the securing member.

The medical securing device may also comprise a storage (not shown in Figures) for receiving number of securing members 102. The storage is advantageously arranged to feed a new securing member 102 when the previous one is introduced to the cardiac implant device 101.

The receiving member 104 can be a piston type, for example, such as is described e.g. in Figures 6A, 6B, 7A, 7B, 8A, 8B, 9A-9H. The piston type receiving member 104 can be operated e.g. by operating, such as pulling an operation member 114, which is advantageously comprised by or in the proximal end 103A of the first stem 103. The operation member 114 is advantageously configured for moving the distal end 103B of the first stem 103, or especially the receiving member 104 towards the cardiac implant device 101 and thereby introducing the securing member 102 to the cardiac implant device 101. Most advantageously the operation member 114 moves the piston type receiving member 104 so that the securing device 102 is outputted at least partially from the distal end 103B or from the hollow structure 106 and against the cardiac implant device 101, after which and advantageously during the same movement the second stem 107 is pulled thereby clamping the cardiac implant device 101 and after this introducing the securing device 102 further and around or into the contact with cardiac implant device 101 and thereby securing said cardiac implant device 101 into the annulus 20.

The operation member 114 advantageously together with the first and/or second stems 103, 107 is/are arranged so that during an operation the following operations are configured to happen either simultaneously or in sequentially by the same operation or movement of the operation member 114:
- the movement of the distal ends 103B, 107B of the first and second stems 103, 107 in relation to each other and towards to each other (pull e.g. the second stem) in their longitudinal direction 108 and thereby to clamp cardiac implant device 101 between the distal ends 103B, 107B of the first and second stems 103, 107,
- the movement of the distal end 103B of the first stem 103, and/or the movement of the receiving member 104 towards the cardiac implant device 101,
- positioning the cardiac implant device 101 in the securing position so i.e. pulling or pushing the cardiac implant device 101 or its lower or upper portion around or over the annulus 20 in its proper securing position, and/or
- introduction the securing member 102 to the cardiac implant device 101 to secure the cardiac implant device 101 to the annulus 20 of the valve with the securing member 102.

As can be seen in Figures the cardiac implant device 101 comprises the upper and/or lower portion 101A, 101B, which are advantageously loop-shaped portions. They may form a coil-shape portion so that during an insertion the cardiac implant device 101 can be rotated into the position. In an advantageous embodiment at least one of the portions 101A, 101B is an open shaped, such as a C or D shaped portion.

## Claims

1. A medical securing device (100) for securing a cardiac implant device (40, 101) with a securing member (102) into an annulus (20) of a heart valve (18) comprised of valve tissue including the annulus and a plurality of leaflets (22, 24), where the cardiac implant device (101) comprises upper and/or lower portion (42, 44, 101A, 101B), whereby, in use, a portion (21) of the valve tissue is coupled with the upper or lower portion, or trapped between the upper and lower portions of the cardiac implant (40, 101), wherein the medical securing device (100) comprises:
- a first elongated stem (103) and a second elongated stem (107) adjacent to the first stem, both having proximal (103A, 107A) and distal (103B, 107B) ends, said first (103) and second (107) stems are configured to be moved in relation to each other in their longitudinal direction (108),
where
- the distal end (103B) of the first stem (103) has a receiving member (104) for receiving the securing member (102),
- whereupon during a movement (105) the distal end (103B) of the first stem and the receiving member (104) in relation to each other, the distal end (103B) of the first stem with the receiving member (104) is configured to introduce the securing member (102) to the cardiac implant device (101) to secure the cardiac implant device (101) to the annulus (20) of the valve with the securing member (102),
**characterized in that**
- the distal end (107B) of the second stem (107) has a counterpart portion (119) for the distal end (103B) of the first stem (103) and/or for the receiving member (104) of the first stem, and
- the counterpart portion (119) comprises an anvil (115) for receiving and bending the securing device (102) at least partially around or partially into the cardiac implant device (101) and/or to the annulus (20) of the valve during the movement (108) of the distal end (103B) of the first stem (103) towards the cardiac implant device (101).

2. The medical securing device of claim 1, wherein the distal end of the first stem has a hollow structure (106), and where the receiving member (104) is arranged to the distal end in a movable manner in relation to the cavity so that during the movement the receiving member is configured to introduce the securing member from the cavity of the distal end to the cardiac implant device to secure the cardiac implant device to the annulus of the valve with the securing member.

3. The medical securing device of any of previous claims, wherein
- 119the distal end (107B) of the second stem (107) is configured to be introduced to the opposite portion of the valve tissue than the distal end (103B) of the first stem (103) and to produce counterforce via the counterpart portion (119) to the distal end of the first stem and thereby clamping the cardiac implant device (101) between the distal ends (103B, 107B) of the first and second stems (103, 107) when the distal end of the first and/or second stems is/are moved in the longitudinal direction (108) towards the cardiac implant device (101), and wherein in addition during the movement (108) the distal end (103B) of the first stem (103) with the receiving member (104) is configured to introduce the securing member (102) to the cardiac implant device (101) to secure the cardiac implant device (101) to the annulus (20) of the valve with the securing member (102).

4. The medical securing device of claim 3, wherein the mutual design of the distal end portions (103B, 107B) of the first and second stems (103, 107) are so that a space (110) is formed between the distal end portions of the first and second stems, whereupon, in use, the space is configured to receive at least portion of the valve tissue and/or leaflets (18).

5. The medical securing device of any of claims 3-4, wherein the second stem (107) has a protrusion portion (111) in the distal end portion (107B) forming the space (110) between the first and second stems (103, 107).

6. The medical securing device of any of previous claims 3-5, wherein the distal end (107B) of the second stem has an angle (112), such as inclined or rectangular portion, towards the extension line (113) of the distal end (103B) of the first stem (103) so that the counterpart portion (119) of the second stem (107) locates essentially in the extension line (113) in the longitudinal direction (108) of the first stem distal end portion (103B).

7. The medical securing device of any of previous claims, especially of claims 2-6, wherein the proximal end (103A) of the first stem (103) comprises an operation member (114) for moving the distal end (103B) of the first stem (103), or especially the receiving member (104) towards the cardiac implant device (101) and thereby introducing the securing member (102) to the cardiac implant device (101).

8. The medical securing device of any of previous claims, wherein the proximal end (103A) of the first stem (103) comprises an operation member (114) during an operation of which the following operations are configured to happen either simultaneously or in sequentially by the same operation of the operation member (114):
- the movement of the distal ends (103B, 107B) of the first and second stems (103, 107) in relation to each other and towards to each other in their longitudinal direction (108) and thereby to clamp cardiac implant device (101) between the distal ends (103B, 107B) of the first and second stems (103, 107),
- the movement of the distal end (103B) of the first stem (103), and/or the movement of the receiving member (104) towards the cardiac implant device (101),
- positioning the cardiac implant device (101) in the securing position, and/or
- introduction of the securing member (102) to the cardiac implant device (101) to secure the cardiac implant device (101) to the annulus (20) of the valve with the securing member (102).

9. The medical securing device of any of claims 7-8, wherein the first stem (103) has a conduit via which the operation member (114) is functionally coupled with the receiving member (104).

10. The medical securing device of any of previous claims, wherein the distal end (103B) of the first stem (103) or the receiving member (104) is configured to press and/or bend a first end (102A) of the securing member at least partially around or partially into the upper and/or lower portion (101A, 101B) of the cardiac implant device (101), when the distal end (103B) of the first stem (103) is pressed against the counterpart portion (119) of the second stem (107).

11. The medical securing device of any of previous claims, wherein the distal end (103B) of the first stem (103) and/or the counterpart portion (119) of the second stem (107) is/are configured to press and/or bend a second end (102B) of the securing member (102) at least partially around or partially into the lower portion (101B) of the cardiac implant device (101) and/or into the annulus (20) of the valve, when the distal end (103B) of the first stem (103) is pressed against the counterpart portion (119) of the second stem (107).

12. 119The medical securing device of any of previous claims, wherein the distal end (103B) of the first stem (103) with the receiving member (104) is configured to introduce the securing device (102) at least partially through or via the cardiac implant device (101) into the annulus (20) of the valve during the movement (108) of the distal end (103B) of the first stem (103) and/or the receiving member (104) in relation the cardiac implant device (101).

13. The medical securing device of any of previous claims, wherein the distal end (103B) of the first stem (103) comprises a recess (116) for supporting the distal end (103B) of the first stem (103) to the upper portion (101A) of the cardiac implant device (101) and/or the distal end (107B) of the second stem (107) comprises a recess (117) for supporting the distal end (107B) of the second stem (107) to the lower portion (101B) of the cardiac implant device (101) during the introduction of the securing member (102).

14. The medical securing device of any of previous claims, wherein the medical securing device (100) comprises a storage for receiving number of securing members (102), whereupon the medical securing device (100) is arranged to feed a new securing member (102) from the storage when the previous one is introduced to the cardiac implant device (101).

15. The medical securing device of any of previous claims, wherein the medical securing device (100) is for an open-heart-operation.

16. The medical securing device of any of previous claims, wherein the securing member (102) is one of the following:
- staple the one end of which is bendable at least partially around or into the cardiac implant device under the pressing force induced by the distal end of the first stem and/or counterpart portion of the second stem,
- staple having at least one hook-shaped end to be introduced at least partially around or into the cardiac implant device (101),
- helical clip,
- locking clip,
- pointed screw,
- spring clip
- skin staple, or
- circular clip.

17. The medical securing device of any of previous claims, wherein the securing member comprises shape memory material, metal or polymer.

18. The medical securing device of any of previous claims, wherein the cardiac implant device (101) comprises the upper and/or lower portion (101A, 101B), which are advantageously loop-shaped portions arranged to form a coil-shape so that during an insertion the cardiac implant device (101) is rotated into the position, and wherein at least one of the portion (101A, 101 B) is at least partially open shaped, such as a C or D shaped.

## Patentansprüche

1. Medizinische Sicherungsvorrichtung (100) zur Sicherung einer Herzimplantatvorrichtung (40, 101) mit einem Befestigungselement (102) in einem Ring (20) einer Herzklappe (18), bestehend aus Klappengewebe, umfassend den Ring und eine Mehrzahl von Segeln (22, 24), wobei die Herzimplantatvorrichtung (101) einen oberen und/oder einen unteren Abschnitt (42, 44, 101A, 101B) umfasst, wodurch in der Verwendung ein Abschnitt (21) des Klappengewebes mit dem oberen oder dem unteren Abschnitt gekoppelt wird, oder zwischen den oberen und den unteren Abschnitten des Herzimplantats (40, 101) eingeklemmt wird, wobei die medizinische Sicherungsvorrichtung (100) umfasst:
- einen ersten lang gestreckten Stab (103) und einen zweiten lang gestreckten Stab (107), der neben dem ersten Stab liegt, wobei beide proximale (103A, 107A) und distale (103B, 107B) Enden aufweisen, wobei der erste (103) und der zweite (107) Stab konfiguriert sind, um relativ zueinander in ihrer Längsrichtung (108) bewegt zu werden,
wobei
- das distale Ende (103B) des ersten Stabs (103) ein Aufnahmeelement (104) zur Aufnahme des Befestigungselements (102) aufweist,
- woraufhin während einer Bewegung (105) des distalen Endes (103B) des ersten Stabs und des Aufnahmeelements (104) relativ zueinander, das distale Ende (103B) des ersten Stabs mit dem Aufnahmeelement (104) konfiguriert wird, um das Befestigungselement (102) in die Herzimplantatvorrichtung (101) einzuführen, um die Herzimplantatvorrichtung (101) an dem Ring (20) des Klappe mit dem Befestigungselement (102) zu sichern,
**dadurch gekennzeichnet, dass**
- das distale Ende (107B) des zweiten Stabs (107) ein Gegenstückteil (119) für das distale Ende (103B) des ersten Stabs (103) und/oder für das Aufnahmeelement (104) des ersten Stabs aufweist, und
- das Gegenstückteil (119) einen Amboss (115) zum Aufnehmen und Biegen des Befestigungselements (102) zumindest zum Teil um oder zum Teil in die Herzimplantatvorrichtung (101) und/oder zu dem Ring (20) der Klappe während der Bewegung (108) des distalen Endes (103B) des ersten Stabs (103) in Richtung der Herzimplantatvorrichtung (101) umfasst.

2. Medizinische Sicherungsvorrichtung nach Anspruch 1, wobei das distale Ende des ersten Stabs eine Hohlraumstruktur (106) aufweist, und wobei das Aufnahmeelement (104) zu dem distalen Ende in einer verschiebbaren Weise relativ zu dem Hohlraum angeordnet ist, sodass während der Bewegung das Aufnahmeelement konfiguriert wird, um das Befestigungselement aus dem Hohlraum des distalen Endes in die Herzimplantatvorrichtung einzuführen, um die Herzimplantatvorrichtung an dem Ring der Klappe mit dem Befestigungselement zu sichern.

3. Medizinische Sicherungsvorrichtung nach einem der vorangehenden Ansprüche, wobei
- das distale Ende (107B) des zweiten Stabs (107) konfiguriert ist, um in den gegenüberliegenden Teil des Klappengewebes zu dem distalen Ende (103B) des ersten Stamms (103) eingeführt zu werden und um eine Gegenkraft mittels des Gegenstückteils (119) auf das distale Ende des ersten Stabs zu erzeugen und dadurch die Herzimplantatvorrichtung (101) zwischen den distalen Enden (103B, 107B) des ersten und des zweiten Stabs (103, 107) festzuklammern, wenn das distale Ende des ersten und/oder des zweiten Stabs in Längsrichtung (108) in Richtung der Herzimplantatvorrichtung (101) bewegt wird, und wobei außerdem während der Bewegung (108) das distale Ende (103B) des ersten Stabs (103) mit dem Aufnahmeelement (104) konfiguriert wird, um das Befestigungselement (102) in die Herzimplantatvorrichtung (101) einzuführen, um die Herzimplantatvorrichtung (101) an dem Ring (20) der Klappe mit dem Befestigungselement (102) zu sichern.

4. Medizinische Sicherungsvorrichtung nach Anspruch 3, wobei die gemeinsame Formgebung der distalen Endabschnitte (103B, 107B) des ersten und des zweiten Stabs (103, 107) derart ist, dass ein freier Raum (110) zwischen den distalen Endabschnitten des ersten und des zweiten Stabs gebildet wird, woraufhin der freie Raum in der Verwendung konfiguriert wird, um zumindest einen Teil des Klappengewebes und/oder der Flügel (18) aufzunehmen.

5. Medizinische Sicherungsvorrichtung nach einem der Ansprüche 3 bis 4, wobei der zweite Stab (107) einen vorspringenden Abschnitt (111) in dem distalen Endabschnitt (107B) aufweist, welcher den freien Raum (110) zwischen dem ersten und dem zweiten Stab (103, 107) bildet.

6. Medizinische Sicherungsvorrichtung nach einem der vorangehenden Ansprüche 3 bis 5, wobei das distale Ende (107B) des zweiten Stabs einen Winkel (112), beispielweise einen geneigten oder rechtwinkligen Abschnitt, in Richtung auf die Verlängerungslinie (113) des distalen Endes (103B) des ersten Stabs (103) aufweist, sodass das Gegenstückteil (119) des zweiten Stabs (107) im Wesentlichen in der Verlängerungslinie (113) in Längsrichtung (108) des distalen Endabschnitts (103B) des ersten Stabs lokalisiert ist.

7. Medizinische Sicherungsvorrichtung nach einem der vorangehenden Ansprüche, insbesondere nach Ansprüchen 2-6, wobei das proximale Ende (103A) des ersten Stabs (103) ein Betätigungselement (114) zum Bewegen des distalen Endes (103B) des ersten Stabs (103), oder insbesondere des Aufnahmeelements (104), in Richtung der Herzimplantatvorrichtung (101) umfasst, und dadurch Einführen des Befestigungselements (102) in die Herzimplantatvorrichtung (101).

8. Medizinische Sicherungsvorrichtung nach einem der vorangehenden Ansprüche, wobei das proximale Ende (103A) des ersten Stabs (103) ein Betätigungselement (114) umfasst, wobei während dessen Betätigung die folgenden Betätigungen konfiguriert werden, um entweder gleichzeitig oder der Reihe nach durch dieselbe Betätigung des Betätigungselements (114) zu erfolgen;
- die Bewegung der distalen Enden (103B, 107B) des ersten und des zweiten Stabs (103, 107) relativ zueinander und aufeinander zu in deren Längsrichtung (108), um dadurch die Herzimplantatvorrichtung (101) zwischen den distalen Enden (103B, 107B) des ersten und des zweiten Stabs (103, 107) festzuklemmen,
- die Bewegung des distalen Endes (103B) des ersten Stabs (103) und/oder die Bewegung des Aufnahmeelements (104) in Richtung der Herzimplantatvorrichtung (101),
- Positionieren der Herzimplantatvorrichtung (101) in der Sicherungsposition, und/oder
- Einführen des Befestigungselements (102) in die Herzimplantatvorrichtung (101), um die Herzimplantatvorrichtung (101) an dem Ring (20) der Klappe mit dem Befestigungselement (102) zu sichern.

9. Medizinische Sicherungsvorrichtung nach einem der Ansprüche 7 bis 8, wobei der erste Stab (103) eine Leitung aufweist, über die das Betätigungselement (114) mit dem Aufnahmeelement (104) funktional verkoppelt ist.

10. Medizinische Sicherungsvorrichtung nach einem der vorangehenden Ansprüche, wobei das distale Ende (103B) des ersten Stabs (103) oder des Aufnahmeelements (104) konfiguriert ist, um ein erstes Ende (102A) des Befestigungselements zumindest zum Teil um oder zum Teil in dem oberen und/oder dem unteren Abschnitt (101A, 101B) der Herzimplantatvorrichtung (101) zu pressen und/oder zu biegen, wenn das distale Ende (103B) des ersten Stabs (103) gegen das Gegenstückteil (119) des zweiten Stabs (107) gepresst wird.

11. Medizinische Sicherungsvorrichtung nach einem der vorangehenden Ansprüche, wobei das distale Ende (103B) des ersten Stabs (103) und/oder das Gegenstückteil (119) des zweiten Stabs (107) konfiguriert ist/sind, um ein zweites Ende (102B) des Befestigungselements (102) zumindest zum Teil um oder zum Teil in den unteren Abschnitt (101B) der Herzimplantatvorrichtung (101) und/oder in den Ring (20) der Klappe zu pressen und/oder zu biegen, wenn das distale Ende (103B) des ersten Stabs (103) gegen das Gegenstückteil (119) des zweiten Stabs (107) gepresst wird.

12. Medizinische Sicherungsvorrichtung nach einem der vorangehenden Ansprüche, wobei das distale Ende (103B) des ersten Stabs (103) mit dem Aufnahmeelement (104) konfiguriert ist, um das Befestigungselement (102) zumindest zum Teil durch oder über die Herzimplantatvorrichtung (101) in den Ring (20) der Klappe während der Bewegung (108) des distalen Endes (103B) des ersten Stabs (103) und/oder des Aufnahmeelements (104) in Verbindung mit der Herzimplantatvorrichtung (101) einzuführen.

13. Medizinische Sicherungsvorrichtung nach einem der vorangehenden Ansprüche, wobei das distale Ende (103B) des ersten Stabs (103) eine Einbuchtung (116) zum Abstützen des distalen Endes (103B) des ersten Stabs (103) gegen den oberen Abschnitt (101A) der Herzimplantatvorrichtung (101) umfasst und/oder das distale Ende (107B) des zweiten Stabs (107) eine Einbuchtung (117) zum Abstützen des distalen Endes (107B) des zweiten Stabs (107) gegen den unteren Abschnitt (101B) der Herzimplantatvorrichtung (101) während der Einführung des Befestigungselements (102) umfasst.

14. Medizinische Sicherungsvorrichtung nach einem der vorangehenden Ansprüche, wobei die medizinische Sicherungsvorrichtung (100) einen Speicher zur Aufnahme einer Anzahl von Befestigungselementen (102) umfasst, woraufhin die medizinische Sicherungsvorrichtung (100) zum Vorschieben eines neuen Befestigungselements (102) aus dem Speicher, wenn das Vorangegangene in die Herzimplantatvorrichtung (101) eingeführt ist, angeordnet wird.

15. Medizinische Sicherungsvorrichtung nach einem der vorangehenden Ansprüche, wobei die medizinische Sicherungsvorrichtung (100) für eine Operation am offenen Herzen ist.

16. Medizinische Sicherungsvorrichtung nach einem der vorangehenden Ansprüche, wobei das Befestigungselement (102) eines der folgenden ist:
- Klammer, deren eines Ende zumindest zum Teil um oder in die Herzimplantatvorrichtung unter der Presskraft, die durch das distale Ende des ersten Stabs und/oder das Gegenstückteil des zweiten Stabs verursacht wird, biegbar ist,
- Klammer mit einem hakenförmigen Ende, das zumindest zum Teil um oder in die Herzimplantatvorrichtung (101) einzuführen ist,
- Spiralklemme,
- Halteklemme,
- spitzzulaufende Schraube,
- Federklemme,
- Hautklammer oder
- kreisförmige Klemme.

17. Medizinische Sicherungsvorrichtung nach einem der vorangehenden Ansprüche, wobei das Befestigungselement ein Formgedächtnismaterial, -metall oder -polymer umfasst.

18. Medizinische Sicherungsvorrichtung nach einem der vorangehenden Ansprüche, wobei die Herzimplantatvorrichtung (101) den oberen und/oder den unteren Abschnitt (101A, 101B) umfasst, die vorteilhafterweise schlaufenförmige Abschnitte sind, die zur Bildung einer Spulenform angeordnet sind, sodass während einer Einführung die Herzimplantatvorrichtung (101) in die Position gedreht wird, und wobei zumindest einer der Abschnitte (101A, 101B) zumindest zum Teil offenkonturig, wie etwa C- oder D-förmig, ist.

## Revendications

1. Dispositif de fixation médicale (100) pour la fixation d'un dispositif d'implant cardiaque (40, 101) avec un élément de fixation (102) dans un anneau (20) d'une valve cardiaque (18) composée de tissu de valve incluant l'anneau et une pluralité de feuillets (22, 24), le dispositif d'implant cardiaque (101) comprenant des sections supérieure et/ou inférieure (42, 44, 101A, 101B), dans lequel, en cours d'utilisation, une section (21) du tissu de valve est couplée à la section supérieure ou inférieure, ou prise entre les sections supérieure et inférieure de l'implant cardiaque (40, 101), le dispositif de fixation médicale (100) comprenant :
- une première tige allongée (103) et une seconde tige allongée (107) adjacente à la première tige, toutes deux comportant des extrémités proximales (103A, 107A) et distales (103B, 107B), lesdites première (103) et seconde (107) tiges étant conçues pour être déplacées l'une par rapport à l'autre dans leur sens longitudinal (108),
- l'extrémité distale (103B) de la première tige (103) comportant un élément récepteur (104) pour recevoir l'élément de fixation (102),
- sur quoi, pendant un mouvement (105), l'extrémité distale (103B) de la première tige et l'élément récepteur (104) l'un par rapport à l'autre, l'extrémité distale (103B) de la première tige comportant l'élément récepteur (104) est conçue pour introduire l'élément de fixation (102) dans le dispositif d'implant cardiaque (101) afin de fixer le dispositif d'implant cardiaque (101) à l'anneau (20) de la valve avec l'élément de fixation (102),
**caractérisé en ce que**
- l'extrémité distale (107B) de la seconde tige (107) comporte une section de contrepartie (119) pour l'extrémité distale (103B) de la première tige (103) et/ou pour l'élément récepteur (104) de la première tige, et
- la section de contrepartie (119) comprend une enclume (115) destinée à recevoir et à courber le dispositif de fixation (102) au moins partiellement autour ou partiellement dans l'intérieur du dispositif d'implant cardiaque (101) et/ou l'anneau (20) de la valve pendant le mouvement (108) de l'extrémité distale (103B) de la première tige (103) en direction du dispositif d'implant cardiaque (101).

2. Dispositif de fixation médicale selon la revendication 1, dans lequel l'extrémité distale de la première tige a une structure une creuse (106), et l'élément récepteur (104) est disposé sur l'extrémité distale de manière mobile par rapport à la cavité de sorte que, pendant le mouvement, l'élément récepteur est conçu pour introduire un élément de fixation depuis la cavité de l'extrémité distale dans le dispositif d'implant cardiaque afin de fixer le dispositif d'implant cardiaque à l'anneau de la valve avec l'élément de fixation.

3. Dispositif de fixation médicale selon l'une quelconque des revendications précédentes, dans lequel
- l'extrémité distale (107B) de la seconde tige (107) est conçue pour être introduite dans la section du tissu de valve opposée à l'extrémité distale (103B) de la première tige (103) et pour produire une contre-force par l'intermédiaire de la section de contrepartie (119) de l'extrémité distale de la première tige et pour serrer ainsi le dispositif d'implant cardiaque (101) entre les extrémités distales (103B, 107B) des première et/ou seconde tiges (103, 107) lorsque l'extrémité distale des première et/ou seconde tiges est déplacée dans le sens longitudinal (108) en direction du dispositif d'implant cardiaque (101), et dans lequel, de plus, pendant le mouvement (108), l'extrémité distale (103B) de la première tige (103) comportant l'élément récepteur (104) est conçue pour introduire l' longitudinal élément de fixation (102) dans le dispositif d'implant cardiaque (101) afin de fixer le dispositif d'implant cardiaque (101) à l'anneau (20) de la valve avec l'élément de fixation (102).

4. Dispositif de fixation médicale selon la revendication 3, dans lequel la conception mutuelle des sections d'extrémités distales (103B, 107B) des première et seconde tiges (103, 107) est telle qu'un espace (110) est formé entre les sections d'extrémités distales des première et seconde tiges en, sur quoi, en cours d'utilisation, l'espace est conçu pour recevoir au moins une partie du tissu de valve et/ou des feuillets (18) .

5. Dispositif de fixation médicale selon l'une quelconque des revendications précédentes 3 à 4, dans lequel la seconde tige (107) a une section en saillie (111) dans la section d'extrémité distale (107B) formant l'espace (110) entre les première et seconde tiges (103, 107).

6. Dispositif de fixation médicale selon l'une quelconque des revendications précédentes 3 à 5, dans lequel l'extrémité distale (107B) de la seconde tige décrit un angle (112), comme une section inclinée ou rectangulaire, en direction de la ligne d'extension (113) de l'extrémité distale (103B) de la première tige (103), de sorte que la section de contrepartie (119) de la seconde tige (107) se situe substantiellement dans la ligne d'extension (113) dans le sens longitudinal (108) de la section terminale distale de la première tige (103B).

7. Dispositif de fixation médicale selon l'une quelconque des revendications précédentes, spécialement selon les revendications 2 à 6, dans lequel l'extrémité proximale (103A) de la première tige (103) contre un élément d'actionnement (114) pour déplacer l'extrémité distale (103B) de la première tige (103), ou spécialement l'élément récepteur (104) en direction du dispositif d'implant cardiaque (101) et introduire ainsi l'élément de fixation (102) dans le dispositif d'implant cardiaque (101).

8. Dispositif de fixation médicale selon l'une quelconque des revendications précédentes, dans lequel l'extrémité proximale (103A) de la première tige (103) comprend un élément d'actionnement (114) pendant un actionnement duquel les opérations suivantes sont conçues pour se produire soit simultanément, soit séquentiellement sous l'effet du même actionnement de l'élément d'actionnement (114) :
- le mouvement des extrémités distales (103B, 107B) des première et seconde tiges (103, 107) l'une par rapport à l'autre et l'une vers l'autre dans leur sens longitudinal (108) pour ainsi serrer le dispositif d'implant cardiaque (101) entre les extrémités distales (103B, 107B) des première et seconde tiges (103, 107),
- le mouvement de l'extrémité distale (103B) de la première tige (103), et/ou le mouvement de l'élément récepteur (104) en direction du dispositif d'implant cardiaque (101),
- positionnement du dispositif d'implant cardiaque (101) dans la position de fixation, et/ou
- introduction de l'élément de fixation (102) dans le dispositif d'implant cardiaque (101) afin de fixer le dispositif d'implant cardiaque (101) à l'anneau (20) de la valve avec l'élément de fixation (102).

9. Dispositif de fixation médicale selon l'une quelconque des revendications 7 à 8, dans lequel la première tige (103) comporte une conduite par laquelle l'élément d'actionnement (114) est couplé fonctionnellement à l'élément récepteur (104).

10. Dispositif de fixation médicale selon l'une quelconque des revendications précédentes, dans lequel l'extrémité distale (103B) de la première tige (103) ou de l'élément récepteur (104) est conçue pour comprimer et/ou courber une première extrémité (102A) de l'élément de fixation au moins partiellement autour ou partiellement dans la section supérieure et/ou inférieure (101A, 101B) du dispositif d'implant cardiaque (101) lorsque l'extrémité distale (103B) de la première tige (103) est comprimée contre la section de contrepartie (119) de la seconde tige (107).

11. Dispositif de fixation médicale selon l'une quelconque des revendications précédentes, dans lequel l'extrémité distale (103B) de la première tige (103) et/ou la section de contrepartie (119) de la seconde tige (107) est/sont conçue(s) pour comprimer et courber une seconde extrémité (102B) de l'élément de fixation au moins partiellement autour ou partiellement dans la section inférieure (101B) du dispositif d'implant cardiaque (101), et/ou dans l'anneau (20) de la valve lorsque l'extrémité distale (103B) de la première tige (103) est comprimée contre la section de contrepartie (119) de la seconde tige (107) .

12. Dispositif de fixation médicale selon l'une quelconque des revendications précédentes, dans lequel l'extrémité distale (103B) de la première tige (103) comportant l'élément récepteur (104) est conçue pour introduire le dispositif de fixation (102) au moins partiellement à travers ou par l'intermédiaire du dispositif d'implant cardiaque (101) dans l'anneau (20) de la valve pendant le mouvement (108) de l'extrémité distale (103B) de la première tige (103) et/ou de l'élément récepteur (104) par rapport au dispositif d'implant cardiaque (101).

13. Dispositif de fixation médicale selon l'une quelconque des revendications précédentes, dans lequel l'extrémité distale (103B) de la première tige (103) comprend un retrait (116) destiné à supporter l'extrémité distale (103B) de la première tige (103) sur la section supérieure (101A) du dispositif d'implant cardiaque (101) et/ou l'extrémité distale (107B) de la seconde tige (107) comprend un retrait (117) destiné à supporter l'extrémité distale (107B) de la seconde tige (107) sur la section inférieure (101B) du dispositif d'implant cardiaque (101) pendant l'introduction de l'élément de fixation (102).

14. Dispositif de fixation médicale selon l'une quelconque des revendications précédentes, dans lequel le dispositif de fixation médicale (100) comprend un rangement pour recevoir nombre d'éléments de fixation (102), sur quoi le dispositif de fixation médicale (100) est conçu pour apporter un nouvel élément de fixation (102) en provenance du rangement lorsque le précédent est introduit dans le dispositif d'implant cardiaque (101).

15. Dispositif de fixation médicale selon l'une quelconque des revendications précédentes, dans lequel le dispositif de fixation médicale (100) est destiné à une opération à cœur ouvert.

16. Dispositif de fixation médicale selon l'une quelconque des revendications précédentes, dans lequel l'élément de fixation (102) est l'un des suivants :
- une agrafe dont une extrémité peut être courbée au moins partiellement autour ou dans le dispositif d'implant cardiaque sous la force de compression induite par l'extrémité distale de la première tige et/ou de la section de contrepartie de la seconde tige,
- une agrafe comportant au moins une extrémité en forme de crochet destinée à être introduite au moins partiellement autour du ou dans le dispositif d'implant cardiaque (101),
- un clip hélicoïdal,
- un clip de verrouillage,
- une vis pointée,
- un clip à ressort,
- une agrafe cutanée, ou
- un clip circulaire.

17. Dispositif de fixation médicale selon l'une quelconque des revendications précédentes, dans lequel l'élément de fixation comprend un matériau, un métal ou un polymère à mémoire de forme.

18. Dispositif de fixation médicale selon l'une quelconque des revendications précédentes, dans lequel le dispositif d'implant cardiaque (101) comprend la section supérieure et/ou inférieure (101A, 101B) qui sont avantageusement des sections en forme de boucles conçues pour former une forme de boucle de manière à ce que, pendant une insertion, le dispositif d'implant cardiaque (101) soit tourné dans la position, au moins une des sections (101A, 101B) ayant au moins partiellement une forme ouverte, telle qu'une forme de C ou de D.
